# DEMANDE DE BREVET EUROPEEN

(11) **EP 2 228 050 A1**
(43) Date de publication de la demande: **15.09.2010**
(21) Numéro de dépôt: 09003372.1
(22) Date de dépôt: 09.03.2009
(51) Int. Cl.: A61K 8/44, A61Q 19/02

(54) **Utilisation de l'acide gamma-aminobutyrique en tant qu'agent dépigmentant**

(71) Demandeur: Cohen, Marcel, 75016 Paris (FR)
(72) Inventeur: Cohen, Marcel, 75016 Paris (FR)
(74) Mandataire: Burtin, Jean-François

(57) **Abrégé**

L'invention se rapporte au domaine de la cosmétique et notamment à l'entretien de la peau et des phanères.

Elle a plus particulièrement pour objet l'utilisation sous forme de compositions cosmétiques de l'acide gamma-aminobutyrique ou d'un de ses sels avec un acide minéral ou organique ou d'un de ses dérivés comme un ester dans des compositions cosmétiques à la concentration de 0,4 à 10% seul ou associé à un extrait de plante riche en polyphénols ou en tannins et/ou à un acide alpha-hydroxylé dans un véhicule aqueux ou huileux.

Utilisation comme agent dépigmentant à raison de 1 à 4 applications par jour pour la peau ou les téguments.

## Description

La présente invention se rapporte aux nécessités de la vie et plus particulièrement aux soins corporels.

Elle a plus précisément pour objet des compositions cosmétiques destinées à assurer un éclaircissement de la peau notamment dans les cas de défauts esthétiques liés à une pigmentation irrégulière anormale ou excessive ou destinées à assurer l'éclaircissement d'une peau normale visant à passer d'un phénotype foncé à un phénotype plus clair.

En outre, les compositions selon l'invention trouvent une utilisation chez les sujets à peau claire pour atténuer les effets du chloasma ou des éphélides.

La présente invention a spécifiquement pour objet l'utilisation d'acide gamma-aminobutyrique (GABA) ou de ses sels ou de ses dérivés en vue de la réalisation de compositions dépigmentantes destinées à être appliquées sur la peau et sur les téguments.

L'acide gamma-aminobutyrique a déjà trouvé des utilisations en dermatologie, notamment comme agent réparateur des tissus. En outre, dans la demande de brevet PCT/FR96/01051, la Demanderesse a déjà décrit l'utilisation de l'acide gamma-aminobutyrique en association avec un acide α-hydroxylé et un extrait de plantes. Comme agent dépigmentant, cette mention est très succinte et ne fournit aucune précision sur la réalité de cette action. Elle n'est d'ailleurs pas l'objet de revendications.

On a maintenant déterminé que cet acide trouvait un emploi remarquable en tant qu'agent dépigmentant en comparaison avec des agents classiques usuels comme les dérivés de l'hydroquinone (arbutine) ou l'acide kojique, tout en étant moins cytotoxique..

L'acide gamma-aminobutyrique ainsi que ses sels et ses dérivés, utilisé dans de telles compositions a été étudié sur des cellules isolées et notamment sur des cultures de mélanocytes humains normaux. Sur les modèles expérimentaux utilisés, il manifeste une action inhibitrice sur la tyrosinase et entraîne une nette diminution de la synthèse de la mélanine. On constate en particulier une réduction significative du contenu intracellulaire en mélanine des cellules en culture. Les résultats obtenus sont fonction de la dose, ce qui est en faveur d'un effet spécifique de l'acide gamma-aminobutyrique sur ce type de paramètre.

Les compositions selon l'invention contiennent comme principe actif l'acide gamma-aminobutyrique tel quel ou sous forme d'un sel avec un acide minéral ou organique, physiologiquement compatible ou encore sous forme d'ester d'alcoyle inférieur ou de polyol.

Parmi les sels d'acide gamma-aminobutyrique utilisables, on pourra citer le chlorhydrate, le sulfate, le phosphate, l'acétate, le propionate, le citrate, le tartrate, le benzoate, le pidolate, le glucose-phosphate, le méthane sulfonate, ou le p-toluène sulfonate.

Les esters d'acide gamma-aminobutyrique qui peuvent être utilisés dans la mise en oeuvre de l'invention peuvent être un ester méthylique, éthylique, propylique, butylique, hexylique, ou un ester de poly éthylène glycol, un ester de polybutylène glycol ou encore un ester de sucre ou de polyol comme le mannitol, le sorbitol ou le glycérol.

Selon les besoins, ces esters seront solubles dans l'eau ou solubles dans des solvants organiques comme les huiles. Leur choix sera déterminé par la nature de la composition cosmétique dans laquelle ils sont incorporés à titre de principe actif.

Les compositions selon l'invention pourront contenir d'autres principes actifs comme des extraits de plantes riches en polyphénols ou en tannins comme l'extrait de mûrier blanc ou bien des acides α-hydroxylés comme l'acide lactique, ou encore des extraits de fruits comme les extraits de citron ou de pamplemousse, également riches en acides alpha-hydroxylés.

Les compositions à base d'acide gamma-aminobutyrique pourront être additionnées d'agents épaississants, d'agents gélifiants, d'agents émulsionnants, d'agents parfumants, d'agents stabilisants, d'agents conservateurs, d'agents améliorant le toucher ou la fluidité.

On pourra citer à cet égard comme agent émulsionnant le stéarate de polyéthylène glycol ou les esters de sucres comme le sucrose ester.

Comme agent épaississant, on pourra citer des dérivés de la cellulose comme la méthylcellulose, l'éthylcellulose, la β-hydroxyéthylcellulose, la carboxyméthylcellulose ou la carboxyméthylcellulose réticulée comme l'Acdisol®.

Un agent gélifiant sera par exemple un polymère d'acide acrylique ou d'acrylamide, comme les carbomères commercialisés sous la dénomination Carbopol® (Goodrich).

La concentration en acide gamma-aminobutyrique dans les compositions pourra varier de 0.4 à 10% de la composition totale avec une préférence pour de 0.5 à 5%.

Les exemples suivants illustrent l'invention sans toutefois la limiter.

### Exemple 1 : Crème à base de chlorydrate d'acide gamma-aminobutyrique

| | | |
|---|---|---|
| Chlorydrate d'acide gamma-aminobutyrique | | 7.5 g |
| Extrait de mûrier blanc | | 0.5 g |
| Extrait de citron BG | | 2.0 g |
| Cire Lanette® | | 3.75 g |
| Agent émulsionnant | | 0.5 g |
| Eau | qsp | 100 g |

### Exemple 2 : Gel à base d'acide gamma-aminobutyrique

| | |
|---|---|
| Acide gamma-aminobutyrique | 2 g |
| Propylène glycol | 5 g |
| Huile d'amandes douces | 10 g |
| Huile de silicone | 0.1 g |
| Germaben II | 0.02 g |
| Carbopol® 936 | 0.4 g |
| Triéthanolamine | 0.1 g |
| Parfum floral | qs |
| Eau purifiée | 80 g |

### Exemple 3 : Etude de la cytotoxicité des compositions à base d'acide gamma-aminobutyrique.

L'objectif de cette étude a été d'évaluer la cytotoxicité de l'acide gamma-aminobutyrique, de l'extrait de mûrier blanc et de l'extrait de citron pris isolément par rapport à celle d'un acide α-hydroxylé bien connu comme l'acide lactique (composé E).

On effectue la détermination sur des mélanocytes humains normaux obtenus à partir d'un sujet jeune (4 ans). Pour les réalisations des essais, les cellules ont été cultivées jusqu'à l'obtention de mono couches confluentes.

### Incubation des cellules :

Les cellules ont mises en incubation pendant 72 heures en l'absence (essai témoin) ou en présence de concentrations croissantes de composé E ou de chacun des produits essayés. Pour le composé E, on a essayé des concentrations de 0.003 ; 0.03 ; 0.075 ; 0.15 ; 0.5 ; 0.75 ; 1.5 et 3% (v/v) et pour le produit selon l'invention : 0.01 ; 0.1 ; 0.25 ; 0.5 ; 1 ; 2.5 ; 5 ; 10% (v/v).

Le produit essayé a été préparé en diluant directement l'acide gamma-aminobutyrique dans de l'eau ultra pure, en quantité suffisante pour 100% (v/v).

A titre de comparaison, on a préparé une solution à 10% d'acide kojique et une solution d'acide lactique à 30% (composition E).

Les préparations essayées ont été ensuite diluées directement dans le milieu d'incubation des mélanocytes.

### Evaluation des effets :

A la fin de la période d'incubation, la viabilité des cellules a été évaluée par une méthode spectrophotométrique de dosage de l'activité des phosphatases intracellulaires.

Selon la méthode de Yan T. et al. (Anal. Biochem. 24 (1996) 103-108), on dose le p-nitrophénol résultant de la transformation du phosphate de p-nitrophényle par les phosphatases intra-cellulaires des cellules viables.

L'absorbance du p-nitrophénol libéré à 405 nm est directement proportionnelle au nombre des cellules viables présentes dans le puits de culture.

### Résultats :

L'acide lactique (composition E) après 72h d'incubation, diminue significativement le nombre de mélanocytes viables, présents dans les puits de culture. Cet effet est détectable (moins de 80 % des cellules viables encore présentes dans le puits de culture) dès la dose de 0.03 %. L'acide lactique est donc nettement cytotoxique.

L'acide gamma-aminobutyrique ne montre un effet cytotoxique décelable dans les mêmes conditions qu'à partir de la dose de 0.5%. En conséquence la composition dépigmentante selon l'invention contenant de l'acide gamma-aminobutyrique à 10 % ne manifeste d'effet cytotoxique qu'à des doses expérimentales plus élevées (voir figure I, II et III et tableau I).

**Tableau I Etude de la cytotoxicité de l'acide lactique (E) et de la composition à base d'acide gamma-aminobutyrique**

| *Actif E* | | | | | | | | |
|---|---|---|---|---|---|---|---|---|
| **Actif E** **(%, v/v)** | **0,003** | **0,03** | **0,075** | **0,15** | **0,3** | **0,75** | **1,5** | **3** |
| % viabilité (par rapport au témoin) | 87,7 | 78,4 | 75,6 | 74,3 | 7,0 | 2,7 | 0,2 | 1,4 |
| | 76,0 | 70,0 | 74,9 | 62,5 | 6,7 | 2,5 | 0,6 | 0,8 |
| | 78,8 | 71,3 | 61,9 | 60,9 | 4,9 | 1,9 | -0,3 | 0,3 |
| Moyenne | **80,8** | **73,2** | **70,8** | **65,9** | **6,2** | **2,4** | **0,2** | **0,8** |
| s.d. | 6,1 | 4,5 | 7,7 | 7,3 | 1,1 | 0,4 | 0,4 | 0,5 |

| *Composition* | | | | | | | | |
|---|---|---|---|---|---|---|---|---|
| **Composition** **(%, v/v)** | **0,01** | **0,1** | **0,25** | **0,5** | **1** | **2,5** | **5** | **10** |
| % viabilité (par rapport au témoin) | 96,6 | 88,0 | 68,0 | 60,6 | 8,2 | 1,8 | 0,2 | 0,1 |
| | 109,7 | 88,6 | 88,1 | 78,7 | 8,3 | 0,5 | -0,2 | -0,1 |
| | 111,7 | 101,6 | 96,1 | 89,5 | 10,7 | 1,6 | -0,4 | 0,3 |
| Moyenne | **106,1** | **92,7** | **84,1** | **76,3** | **9,1** | **1,3** | **-0,1** | **0,1** |
| s.d. | 8,2 | 7,7 | 14,5 | 14,6 | 1,4 | 0,7 | 0,3 | 0,2 |

Les compositions à base d'extrait de mûrier blanc ou d'extrait de citron ont également des effets modérés.

### Exemple 4 : Effet « mélano-modulateur » des principes actifs contenus dans les compositions selon l'invention

La recherche d'un effet mélanomodulateur a été menée en 2 étapes :
- Effet mélanomodulateur des ingrédients pris séparément aux concentrations prédéterminées dans le test de cytotoxicité.
- Effet mélanomodulateur du complexe, i.e. GABA en association avec les ingrédients mûrier blanc, citron et acide lactique.

### 1^{ère} étape

Ces essais ont été menés sur un modèle de mélanocytes humains normaux cultivés en mono couche.

L'acide gamma-aminobutyrique, l'extrait de citron et l'extrait de mûrier blanc ont été solubilisés et dilués directement dans le milieu de culture.

### Incubation des cellules avec les produits essayés :

Les mélanocytes humains normaux ont été mis en incubation pendant 72 heures en l'absence (témoin) ou en présence d'un produit de référence (acide kojique 250 µM)) ou de concentrations croissantes des produits actifs essayés.

Acide gamma-aminobutyrique (composition A) 0.001 ; 0.01 ; 0.1%
Extrait de mûrier blanc ( Composition C) 0.01 ; 0.1 ; 0.5%
Extrait de citron (Composition D) 0.01 ; 0.1 ; 1%

Dans cette méthode, on dose le rapport mélanine/protéines en pourcentage du témoin. On a déterminé également les valeurs obtenues avec le solvant seul et une solution d'acide kojique à 250 µM dans ce solvant(DMSO) pris comme substance de référence.

Le tableau II fournit les résultats obtenus sur les principes actifs séparés A (acide gamma-aminobutyrique), C, D et l'acide lactique (composition E). L'acide gamma-aminobutyrique fournit des résultats statistiquement significatifs dès la concentration de 0.1%. Les composés C et D ont un effet mélanomodulateur modéré.

### 2^{ème} étape

On a également évalué l'effet d'un complexe contenant les principes actifs A (acide gamma-aminobutyrique), C, D et l'acide lactique (composition E) comme élément de comparaison sur la synthèse de mélanine dans un modèle de mélanocytes humains normaux cultivés en monocouche.

**Tableau II Véhicule (DMSO) et produit de référence**

| | **Contrôle** | | **DMSO** | | **Acide Kojique** **250 µM** | |
|---|---|---|---|---|---|---|
| mélanine / protéines (% du témoin) | 96,8 | 100,6 | 95,6 | 98,2 | 73,1 | 65,9 |
| | 97,8 | 110,7 | 98,4 | 105,4 | 84,3 | 69,5 |
| | 93,0 | 91,4 | 99,4 | 105,9 | 84,5 | 78,1 |
| | 110,5 | 99,2 | 95,7 | 97,3 | 93,9 | 66,3 |
| | 100,6 | 106,4 | 97,2 | 91,5 | 88,0 | 70,6 |
| | 102,4 | 102,9 | - | - | - | - |
| | 100,9 | 99,3 | - | - | - | - |
| | 96,1 | 91.4 | - | - | - | - |
| **Moyenne** | **100,00** | | **98,45** | | **77,40*** | |
| S.D. | 4,56 | | 3,33 | | 4,57 | |

| | | | | | | |
|---|---|---|---|---|---|---|
| * : moyenne significativement différente de celle du groupe témoin «milieu seul» (p<0,05) | | | | | | |

**Actifs à l'essai**

| | **A (%,** **p/v)** | | | **C (%,** **v/v)** | | | **D (%,v/v)** | | |
|---|---|---|---|---|---|---|---|---|---|
| | **0,001** | **0,01** | **0,1** | **0,01** | **0,1** | **0,5** | **0,01** | **0,1** | **1** |
| Mélanines/ protéines (% du témoin) | 98,8 | 87,0 | 74,3 | 104,8 | 138,2 | 162,2 | 94,9 | 105,5 | 133,9 |
| | 108,6 | 101,8 | 76,4 | 106,8 | 147,6 | 160,4 | 104,2 | 94,8 | 134,8 |
| | 98,3 | 101,1 | 74,2 | 99,8 | 154,3 | 155,9 | 98,0 | 104,4 | 130,5 |
| | - | - | 71,3 | - | - | 165,5 | 95,7 | 97,3 | 142,3 |
| | - | - | - | - | - | 181,0 | 97,2 | 91,5 | 122,7 |
| | - | - | - | - | - | 181,1 | - | - | 133,5 |
| | | | | | | 177,2 | - | - | - |
| | - | - | - | - | - | 178,2 | - | - | - |
| **Moyenne** | **101,9** | **96,6** | **74,0*** | **103,8** | **146,7** | **170,2*** | **99,0** | **101,6** | **133,0*** |
| s.d. | 5,8 | 8,4 | 2,1 | 3,6 | 8,1 | 10,3 | 4,8 | 5,9 | 6,4 |

| | | | | | | | | | |
|---|---|---|---|---|---|---|---|---|---|
| * : moyenne significativement différente de celle du groupe témoin "milieu seul" (p<0,05) A : Composition à base d'acide gamma-aminobutyrique C : Composition à base de mûrier blanc D : Composition à base d'extrait de citron | | | | | | | | | |

### Produits à tester :

Les compositions ont été préparées en diluant directement l'acide gamma-aminobutyrique A, les principes C et D et l'acide lactique (composé E) dans de l'eau ultra pure aux concentrations suivantes :
- acide gamma-aminobutyrique 10%
- extrait de mûrier blanc 1 %
- extrait de citron 1 %
- acide lactique 1.5%
- eau ultra pure
   Les compositions à l'essai ont été diluées ensuite directement dans le milieu d'incubation des mélanocytes aux concentrations de 0.1 ; 0.5 ; et de 1% (v/v).

### Système d'essai :

On a utilisé des mélanocytes humains normaux issus d'un sujet jeune âgé de 4 ans. On les a cultivé en monocouche jusqu'à confluence de 80%.

### Produit de référence :

On a employé comme inhibiteur de tyrosinase de référence, l'acide kojique à 250 µM.

### Incubation des cellules :

Les mélanocytes ont été mis en incubation pendant 72 heures à 37°C sous atmosphère humide et sous 5% de CO₂ en l'absence (témoin) ou en présence d'acide kojique ou de concentrations croissantes de principes actifs à l'essai (0.1 ; 0.5 1%)

### Evaluation des effets anti-mélanine :

### 1.Dosage de la mélanine :

A la fin de la période d'incubation, le contenu intracellulaire en mélanine a été quantifié après lyse cellulaire par mesure spectrophotométrique à 405nm.

### 2.Dosage des protéines :

A la fin de la période d'incubation, les protéines contenues dans les lysats cellulaires ont été dosés par une méthode spectrocolorimétrique faisant appel au bleu de Coomassie (selon la méthode de Bradford M. Anal. Biochem. 72 (1976) 248-254).

### 3.Résultats :

Les résultats sont donnés sous forme de pourcentage de mélanine par rapport aux témoins (moyenne + ou- déviation standard SD) calculés à partir de valeurs obtenues en µg de mélanine intracellulaire par mg de protéines totales du tapis cellulaire.

On a déterminé la significativité statistique des différences constatées entre les conditions des « témoin » et « Produits à l'essai » selon une analyse de variance à un seul facteur (ANOVA) suivi d'un test de Holm-Sidak (* = µ < 0,05)

Les résultats obtenus avec la composition à base d'acide gamma-aminobutyrique montrent une réduction significative du contenu intracellulaire en mélanine des cellules en culture.

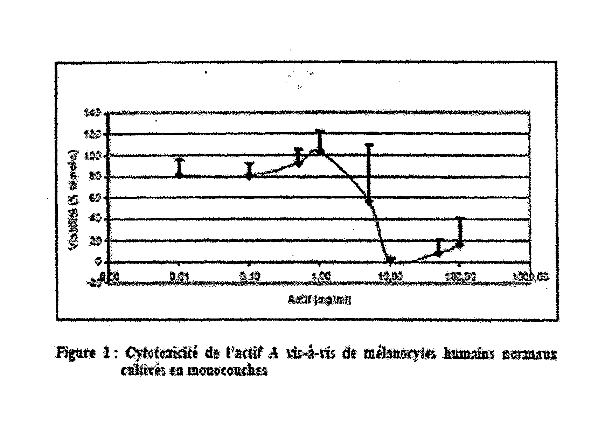

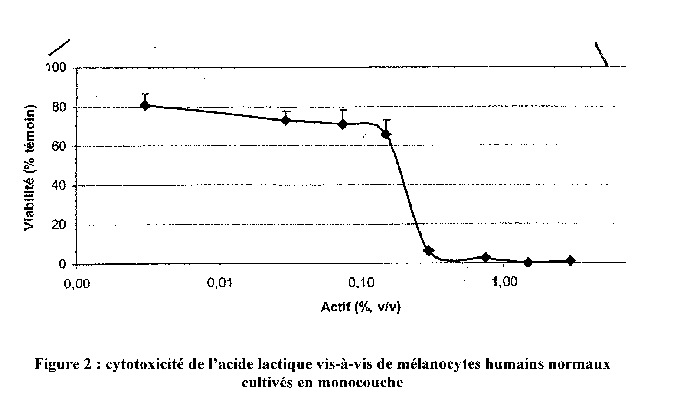

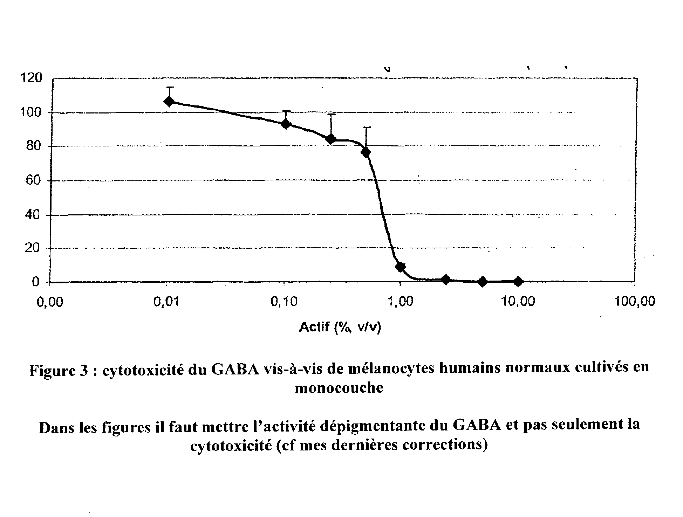

La valeur obtenue avec 0.1% de principe actif n'est pas significative. Les valeurs obtenues avec une concentration à 0.5% (-19.7%) et à 1% (-25.4%) sont statistiquement significatives (p<0.05). Ces résultats sont figurés à la figure 3 et au tableau III.

L'acide kojique à 250 µM utilisé comme inhibiteur de mélanogénèse de référence, à titre de comparaison, produit une diminution significative du contenu intracellulaire en mélanine des cellules mises en culture (réduction de 15.5%) (p < 0.05).

Ce résultat représenté au tableau III fournit la preuve de la validité de la méthode d'investigation.

Les résultats obtenus avec les compositions à base d'acide gamma-aminobutyrique sont dose-dépendants, ce qui est en faveur d'un effet spécifique du complexe sur la paramètre étudié.

### Résultats

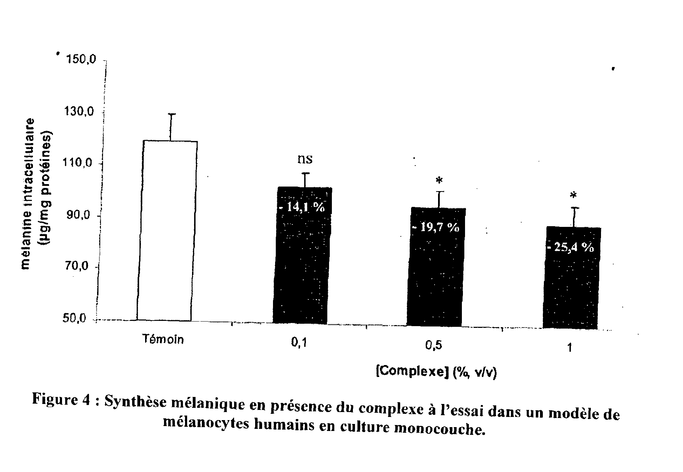

Les résultats présentés dans la figure 4 et au tableau III ci-dessous montrent que dans les conditions expérimentales retenues :

Le complexe à l'essai réduit significativement le contenu intracellulaire en mélanine des cellules en culture :

| | | |
|---|---|---|
| Complexe à 0,1% (v/v) | ___> | - 14,1% (ns) |
| Complexe à 0,5% (v/v) | ___> | - 19,7% (p<0,05) |
| Complexe à 1% (v/v) | ___> | - 25,4% (p<0,05) |

**Tableau III Complexes à l'essai**

| | | **Complexe à l'essai (%, v/v)** | | |
|---|---|---|---|---|
| | **Témoins** | **0,1** | **0,5** | **1** |
| Mélanines/ protéines (% du témoin) | 131,4 | 108,1 | 98,4 | 97,7 |
| | 115,1 | 102,4 | 100,1 | 83,3 |
| | 111,1 | 96,6 | 88,8 | 85,8 |
| | 103,8 | 103,6 | 88,6 | 92,2 |
| | 104,5 | - | - | - |
| **Moyenne** | **119,2** | **102,4** | **95,8*** | **88,9*** |
| s.d. | 10,8 | 5,8 | 6,1 | 7,7 |
| **% témoin** | **100** | **85,9** | **80,3** | **74,6** |

| | | | | |
|---|---|---|---|---|
| * : moyenne significativement différente de celle du groupe témoin "milieu seul" (p<0,05) | | | | |

**Produit de référence : acide kojique 250 µM**

| | **Témoins** | **Acide kojique 250** µ**M** | | |
|---|---|---|---|---|
| Mélanines/ protéines (% du témoin) | 131,4 | 106,3 | | |
| | 115,1 | 93,2 | | |
| | 111,1 | 102,8 | | |
| | 103,8 | 94,1 | | |
| | 104,5 | 97,9 | | |
| **Moyenne** | **119,2** | **100,8*** | | |
| s.d. | 10,8 | 6,8 | | |
| **% témoin** | **100** | **84,5** | | |

| | | | | |
|---|---|---|---|---|
| * : moyenne significativement différente de celle du groupe témoin "milieu seul" (p<0,05) | | | | |

L'acide gamma-aminobutyrique ou ses sels avec un acide minéral ou organique ou ses dérivés est utilisé sous forme de compositions cosmétiques aqueuses ou huileuses choisies parmi les laits, les lotions, les émulsions huile dans eau ou eau dans huile, les crèmes, les gels et les eaux de toilette.

La concentration en acide gamma-aminobutyrique pourra varier dans de larges proportions de 0,4 à 10% en poids de la composition totale. Une concentration davantage préférée varie de 0,5 à 5% en poids.

Lorsque l'acide gamma-aminobutyrique est utilisé sous forme de sel ou de dérivé, les quantités utilisées doivent tenir compte de la teneur en acide gamma-aminobutyrique dans le sel ou le dérivé. Dans le cas d'ester de polyphénol, les concentrations en acide gamma-aminobutyrique doivent être calculées pour réaliser une teneur en principe actif variant de 0,4 à 10% en poids dans les compositions cosmétiques de l'invention.

## Revendications

1. Utilisation de l'acide gamma-aminobutyrique, d'un de ses sels ou d'un de ses esters d'alcoyle inférieur ou de polyol en tant qu'agent dépigmentant ou mélano modulateur.

2. Utilisation selon la revendication 1 dans laquelle on utilise l'acide gamma-aminobutyrique libre comme agent dépigmentant.

3. Utilisation selon la revendication 1 dans laquelle on utilise un sel d'acide gamma-aminobutyrique comme agent dépigmentant.

4. Utilisation selon la revendication 1 dans laquelle le sel d'acide gamma-aminobutyrique est un sel avec un acide minéral ou organique physiologiquement compatible.

5. Utilisation selon la revendication 1 ou la revendication 4 dans laquelle le sel d'acide gamma-aminobutyrique est le chlorydrate d'acide gamma-aminobutyrique.

6. Utilisation selon la revendication 1 dans laquelle on utilise un ester d'alcoyle inférieur d'acide gamma-aminobutyrique comme agent dépigmentant.

7. Utilisation selon la revendication 1 et la revendication 6 dans laquelle on utilise un ester d'alcoyle inférieur d'acide gamma-aminobutyrique soluble dans l'eau ou soluble dans les solvants organiques.

8. Utilisation selon la revendication 1 dans laquelle l'acide gamma-aminobutyrique est additionné d'un extrait végétal riche en polyphénols ou en tannins, comme agent dépigmentant.

9. Utilisation de l'acide gamma-aminobutyrique, de ses sels ou de ses esters d'alcoyle inférieur ou de polyol dans des compositions cosmétiques topiques.

10. Utilisation de l'acide gamma-aminobutyrique selon la revendication 9 dans des compositions aqueuses ou huileuses choisies parmi les laits, les lotions, les émulsions huile dans l'eau ou eau dans l'huile, les crèmes, les gels et les eaux de toilette.

11. Utilisation de l'acide gamma-aminobutyrique, un de ses sels ou un de ses esters selon la revendication 1 dans des compositions cosmétiques à une concentration variant de 0.4% à 10%.

12. Utilisation de l'acide gamma aminobutyrique selon la revendication 11 à une concentration variant de 0.5% à 5% comme agent dépigmentant.
